# EUROPEAN PATENT SPECIFICATION

(11) **EP 1 248 774 B1**
(45) Date of publication and mention of the grant of the patent: **07.10.2009**
(21) Application number: 00984681.7
(22) Date of filing: 06.12.2000
(51) Int. Cl.: C07D 303/14, C07D 303/24, C07D 303/40, A61K 31/355, A61K 31/05, A61K 31/09, A61P 17/06, A61P 29/00

(54) **POLYHYDROXYSTILBENES AND STILBENE OXIDES AS ANTIPSORIATIC AGENTS AND PROTEIN KINASE INHIBITORS**
POLYHYDROXYSTILBENE UND -STILBENOXIDE ALS ANTIPSORIASISMITTEL UND PROTEINKINASEHEMMER
TRAITEMENT ANTI-INFLAMMATOIRE ET PSORIASIQUE ET INHIBITION DE LA PROTEINE KINASE PAR DES HYDROXYLSTILBENES ET DE NOUVEAUX DERIVES DE STILBENE OU ANALOGUES

(30) Priority: 06.12.1999 US 168758 P; 28.12.1999 US 173300 P
(43) Date of publication of application: 16.10.2002
(73) Proprietor: Welichem Biotech Inc., Burnaby, British Columbia V5J 5G6 (CA)
(72) Inventor: CHEN, Genhui, Burnaby, British Columbia V5G 3L1 (CA); WEBSTER, John, M., North Vancouver, British Columbia V7R 4P (CA); LI, Jianxiong, Port Moody, British Columbia V3H 2T4 (CA); HU, Kaiji, Burnaby, British Columbia V5B 2B1 (CA); ZHU, Jiang, Cupertino, California 95014 (US)
(74) Representative: Schüssler, Andrea
(86) International application number: PCT/CA2000/001433
(87) International publication number: WO 2001/042231

(56) References cited:
- EP-A- 0 170 105
- EP-A- 0 558 950
- WO-A-92/16486
- WO-A-92/19583
- WO-A-99/40056
- WO-A-99/59561
- FR-A- 2 785 284
- GB-A- 1 465 661
- US-A- 4 723 028
- US-A- 4 788 304
- US-A- 4 876 381
- US-A- 4 933 365
- US-A- 5 547 983
- US-A- 5 556 996
- US-A- 6 008 260
- PATENT ABSTRACTS OF JAPAN vol. 1997, no. 04, 30 April 1997 (1997-04-30) & JP 08 337523 A (TAIHO YAKUHIN KOGYO K. K.), 24 December 1996 (1996-12-24)
- T. MAJIMA ET AL.: "Cis-Trans Isomerization and Oxidation of Radical Cations of Stilbene Derivatives" J. ORG. CHEM., vol. 61, no. 22, 1996, pages 7793-7800, XP002163396
- PATENT ABSTRACTS OF JAPAN vol. 1995, no. 05, 30 June 1995 (1995-06-30) & JP 07 053359 A (KAO CORP.), 28 February 1995 (1995-02-28)
- PATENT ABSTRACTS OF JAPAN vol. 007, no. 281, 15 December 1983 (1983-12-15) & JP 58 159410 A (MITSUBISHI CHEM. IND., LTD.), 21 September 1983 (1983-09-21)
- M. CUSHMAN ET AL.: "Synthesis and Evaluation of Analogues of (Z)-1-(4-Methoxyphenyl)-2-(3,4,5-trimethox yphenyl)ethene as Potential Cytotoxic and Antimitotic Agents" J. MED. CHEM., vol. 35, no. 12, 1992, pages 2293-2306, XP000571677

## Description

### BACKGROUND OF THE INVENTION

The stilbenes isolated from Photorhabdus species bacteria are known to have antibiotic activity. See V.J. Paul, S. Frautschv, W. Fenical, and K.H. Nealson, Journal of Chemical Ecology, 7: 589-597 (1981), and K. Hu, J. Li, W. Wang, H. Wu, H. Lin and J. M. Webster, Canadian Journal of Microbiology. 44: 1072-1077 (1998). However, these compounds have not been shown to have other biological activity. A similar compound, resveratrol, has been disclosed as having cancer preventive (Jang et al. 1997) and protein kinase C inhibitory (Garcia-Garcia et. al., 1999) activities.

There are many common conditions that cannot be treated successfully by antibiotics. Some of these are inflammatory diseases. The compounds of the invention possess specific anti-inflammatory properties.

Inflammatory diseases, whether of a chronic or acute nature, represent a substantial problem in the healthcare industry. Chronic inflammation is considered to be inflammation of a prolonged duration (weeks or months) in which active inflammation, tissue destruction and attempts at healing are proceeding simultaneously *(*Robbins Pathological Basis of Disease by R. S. Cotran, v. Kumar , and S. L. Robbins, W. B, Saunders Co., p. 75, 1989). Although chronic inflammation can follow an acute inflammatory episode, it can also begin as an insidious process that progresses with time, for example, as a result of a persistent infection ( *e.g.,* tuberculosis, syphilis, fungal infection) which causes a delayed hypersensitivity reaction, prolonged exposure to endogenous *(e.g.*, elevated plasma lipids) or exogenous *(e.g.,* silica, asbestos, cigarette tar, surgical sutures) toxins, or, autoimmune reactions against the body's own tissues *(e.g.*, rheumatoid arthritis, systemic lupus erythematosus, multiple sclerosis, psoriasis). Chronic inflammatory diseases therefore, include many common medical conditions such as rheumatoid arthritis, restenosis, psoriasis, multiple sclerosis, surgical adhesions, tuberculosis, and chronic inflammatory lung and airway diseases *(e.g.*, asthma, pneumoconiosis, chronic obstructive pulmonary disease, nasal polyps and pulmonary fibrosis).

Psoriasis is a common, chronic inflammatory skin disease characterized by rapid multiplication and turnover of the epithelial cells with a consequent thickening of the epidermis, as well as inflamed swollen skin lesions covered with silvery white scaling patches and raised, inflamed, thickened and scaly lesions, which itch, burn, sting and bleed easily. It is therefore a disease characterized not only by inflammation, but also by proliferation of cells. In the respect of proliferation, therefore, it has similarities to cancers, and both psoriasis and cancers can be described as proliferative diseases. In approximately 10% of patients, psoriasis is accompanied by pronounced arthropathic symptoms that are similar to the changes seen in rheumatoid arthritis. Approximately 2 to 3% of the U.S. population suffers from psoriasis, with 250,000 new cases being diagnosed each year. The compounds of the invention possess specific activity against psoriasis.

Antibiotic activity is also not a predictor of utility as a protein kinease inhibitor. Protein kinease is implicated in many diseases, including cancers, as well as being a factor in diabetes, heromatous plaque and epidermal proliferation (including psoriasis.). The compounds of the invention possess specific protein kinase inhibiting activity.

It is believed that protein kinase inhibitors may be of great importance in the control of uncontrolled cellular reproduction, i.e. in cellular reproduction disorders. For example DNA-PK is a serine/threonine protein kinase that is composed of a very large catalytic polypeptide and a DNA binding/targeting regulatory subunit (Ku autoantigen). Ku was first recognized as a heterodimeric (p70/p80) nuclear phosphoprote in that reacted with sera from patients suffering from autoimmune diseases lupus erythematosus and scleroderma polymyositis. Casein kinase II (Ck2) is a serine/threonine kinase that phosphorylates acidic protein such as casein. Ck2 has been shown to play multiple roles inside the cell and can be activated by numereous growth factors, hormones and cytokines. Ck2 has multiple substrate targets inside the cell which are untimately involved in the regulation of DNA, RNA and protein synthesis. Ck2 plays a role in controlling mitogenic signalling, neuritogenesis. Ck2 has been shown to be involved with numerous disease states. Elevated Ck2 levels have been demonstrated in solid human tumors and rapid proliferating non-neoplastic tisue such as colorectal mucosa. Ck2 activity was much hither in metastatic melanoma and in cells transformed by human cytomegalovirus. Infection of animals with protoxoan parasite resulted in fatal lymphoproliferative syndrome that is asssociated with the over expression of Ck2. Ck2 activity has also been demonstrated to be elevated in Alzheimers disease.

Amson et al. (1989) showed that the 33-kD product of the PIM gene is highly expressed in the liver and spleen during fetal hematopoiesis. In contrast, it is only slightly expressed in circulating granulocytes in adults. It was over expressed in hematopoietic malignancies, particularly in myeloid and lymphoidacute leukemias. The results implied a physiologic role of the Pim 1 oncogene during hematopoietic development and a deregulation of the gene in various leukemias. Saris et al. (1991) provided evidence that both the murine and the human Pim1 gene products are protein-serine/threonine kinases. In the mouse, at any rate, they showed that the gene encodes both a 44-and a 34-kD protein, the former being an amino-terminal extension of the latter which is synthesized by alternative translation initiation at an upstream CUG codon. Ark et al. (1991) provided refined mapping of the Pim-1 locus in the mouse and used the Pim-1 gene as a marker for further genetic analysis of t-haplotypes on mouse chromosome 17. To understand the function of Pim-1 and its role in hematopoietic development, Laird et al. (1993) generated mice deficient in Pim-1 function. Pim-1-deficient mice were ostensibly normal, healthy, and fertile; however, detailed analysis demonstrated a correlation of Pim-1 deficiency with erythrocyte microcytosis, whereas over expression of Pim-1 in transgenic mice resulted in erythrocyte macrocytosis.

WO 99/59561 A discloses resveratrol and its derivatives as potent inhibitors of myeloperoxides, an enzyme released by neutrophils, which causes tissue damage in diseases involving chronic inflammation. Such diseases include but are not limited to artherosclerosis, arthritis, Alzheimer's disease, sepsis (endotoxic shock), inflammatory skin disease (psoriasis), inflammatory gum disease (gingivitis), and inflammatory bowel disease.

JP 07053359 A provides piceatannol and its derivatives for the use as an active component, compounded with a carrier and formed in the form of a pharmaceutical preparation to obtain the objective preparation for an anti-inflammatory agent, an anti-allergic agent, etc. having excellent lipoxygenase inhibiting action.

Cushman et al. (1992) synthesized and evaluated a series of stilbenes, i.e. analogues of (Z)-1-(4-Methoxyphenyl)-2-(3,4,5-trimethoxyphenyl)ethene as potential cytotoxic and antimitotic agents by using five human cancer cell lines: A-549 non-small cell lung, MCF-7 breast, HT-29 colon, SKMEL-5 melanoma, and MLM melanoma.

WO 99/40056 A concerns enzymatic aromatic hydroxylation-activated prodrugs, particularly anti-tumor prodrugs and those which are specifically activated by the hydroxylation activity of the enzyme CYP1B1. The intratumoral hydroxylation of the prodrugs provides them with a surprising and unexpected efficacy, whereas their styrene- or calchone sub-structure is essential therefore.

Recent studies on the molecular basis or neoplastic transformation have identified a family of genes, designated oncogenes, whose aberrant expression causes tumorigenesis. For example, the RNA tumour viruses possess such an oncogene sequence whose expression determines neoplastic conversion of infected cells. The tyrosine kinase Lck are expressed primarily in different types of hematopoietic cells. The Lck protein is found in thymocytes and mature T cells and has been reported to be expressed in mature mouse splenic B cells. Campbell et al. (1992). Lck inhibitors are of value in the treatment of a number of such disorders (for example, the treatment of autoimmune diseases), as Lck inhibition blocks T cell activation. The treatment of T cell mediated diseases, including inhibition of T cell activation and proliferation, is a particularly preferred embodiment of the present invention. Compounds which selectively block T cell activation and proliferation are preferred.

Accordingly, a specific inhibitor of these kinases can be useful in investigating the mechanism of cancerogenesis, cell proliferation, differentiations and autoimmunology and it can be effective in prevention and chemotherapy of cancer and other pathological proliferative conditions. Hence the compounds according to the present invention can be useful in the treatment of pathological proliferation and autoimmune disorders in mammals, including humans. A human or animal, e.g. a mammal, can thus be treated by a method comprising the administration thereto of a therapeutically effective amount of one of the compounds of the invention. Amelioration of the disease state or disorder from which the human or animal is suffering can be achieved. Typical examples of such disorders are benign and malignant tumours, including leukaemia such as myeloblastic leukaemia, lymphoma, sarcoma, neuroblastoma, Wilm's tumour, malignant neoplasm of the bladder, breast, lung or thyroid, neoplasias of epithelialorigin, such as mammacarcinoma. Moreover, they can be useful in the treatment of epidermal hyper-proliferation, such aspsoriasis. The compounds of the invention can also be useful in inhibiting the development of the heromatous plaque and restenosis, in the control of angiogenesis, as anti-metastatic agents and in treating diabetic complications. They have also utility in the control of immune system diseases, e.g. as immuno-suppressants, as far as protein tyrosine kinases are involved in these diseases.

Transplant rejection (graft v. host disease) and surgical adhesions are also affected by protein kinases. The protein kinease inhibition and anti-inflamatory properties of the compounds of this inventiongive rise to utility in surgery to reduce transplant rejection and surgical adhesions.

In a particular embodiment, the compounds of the present invention are useful for the treatment of the aforementioned exemplary disorders irrespective of their etiology, for example, for the treatment of transplant rejection, rheumatoid arthritis, multiple sclerosis, chronic obstructive pulmonary disease, inflammatory bowel disease, lupus, graft vs host disease, T-cell mediated hypersensitivity disease, psoriasis, Hashimoto's thyroiditis, Guillain-Barre syndrome, cancer, contact dermatitis,allergic disease such as allergic rhinitis, asthma, ischemic or reperfusion injury, or atopic dermatitis

### SUMMARY OF THE INVENTION

The present invention relates to compositions containing the hydroxylated stilbenes and their derivatives and analogues which are useful in the treatment of inflammatory diseases, for the treatment of psoriasis, and for the inhibition of protein kinases.

The invention also comprises the novel synthesis methods used to make the compounds. These methods render commercial manufacture possible and do not require recovery from insects.

The present invention also comprises novel compounds, of the formula II given below.

### DETAILED DESCRIPTION OF THE INVENTION

In one preferred embodiment, this invention relates to compositions containing an antinflammatory amount of a compound of the formula (Formula I): 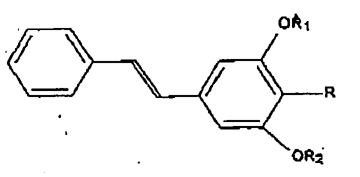 wherein
R is selected from the group consisting of
halogen;
alkyl with carbon between 1 to 18;
alkenyl with carbon between 1 to 18;
R1 and R2 are independently selected from the group consisting of
hydrogen;
alkyl with carbon between 1 to 18;
acyl with carbon between 1 to 18;
and the pharmaceutically acceptable salts thereof.

The compounds of Formula I are particularly useful against psoriasis, and, in a preferred embodiment, the compound is present in an antipsoriatic amount.

The compounds of the invention are also useful as protein kinase inhibitors, and in a preferred embodiment, the compound is present in an amount to inhibit protein kinase activity.

The inventions also comprise methods of treating inflammation, or psoriasis, or treating conditions which are treatable by protein kinease inhibition, by administering a pharaceutically effective amount of the compounds of the invention.

Referred compounds for use in the compositions of the present invention are those compounds wherein R is an alkyl group, R1 and R2 are hydrogen. Particularly preferred are those compounds wherein R are branched short chain alkyl group, R1 and R2 are hydrogen. A highly preferred compound is 3,5-dihydroxy-4-isopropylstilbene.

In another preferred embodiment, R is selected from the group consisting of substituted and unsubstituted alkyl with carbon between 1 to 18, substituted and unsubstituted alkenyl with carbon between 1 to 18, halide,
R1 and R2 are selected from the group of acyl with carbon between 1 to 18, hydrogen and alkyl groups with carbon between 1 to 18,
and the pharmaceutically acceptable salts thereof.

In a further preferred embodiment, R is selected from the group consisting of substituted and unsubstituted alkyl with carbon between 1 to 18, substituted and unsubstituted alkenyl with carbon between 1 to 18, and halide
and
R1 and R2 are selected from the group of acyl with carbon between 1 to 18, hydrogen and methyl groups,
and the pharmaceutically acceptable salts thereof.

In a particularly preferred embodiment, the composition contains 3,5-dihydroxy-4-*iso*propylstilbene.

This invention also relates to novel compounds and compositions containing an anti-inflammatory amount of a compound of the formula II : 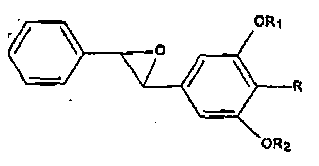 wherein:
R is selected from the group consisting of
   hydrogen;
   halogen;
   alkyl with carbon between 1 to 18;
   alkenyl with carbon between 1 to 18;
   alkoxy with carbon between 1 to 18;
R1 and R2 are independently selected from the group consisting of
   hydrogen;
   alkyl with carbon between 1 to 18;
   acyl with carbon between 1 to 18;
   or pharmaceutically acceptable salts thereof.

In addition to the free bases of formula I and II, the pharmaceutically acceptable salts and the like are contemplated as being within the scope of this invention

The compounds of this invention and the inventive compositions, encompass both the trans and cis stereochemical forms of the compounds,and mixtures of the trans and cis forms.

### BRIEF DESCRIPTION OF THE DRAWINGS

Figure 1 is a graph showing the effect of 3,5-dihydroxy-4-isopropylstilbene on crystal induced neutrophil activation.
Figure 2 is a graph showing the effect of 3,5-dihydroxy-4-isopropylstilbene on fMLP induced neutrophil activation.

### Example 1 - Synthesis of the Inventive Compounds

The compounds useful in the compositions of the present invention may be prepared from 3,4-dihydroxybenzoic acid and 4-bromo-3,4-dihydroxybenzoic acid of the formula

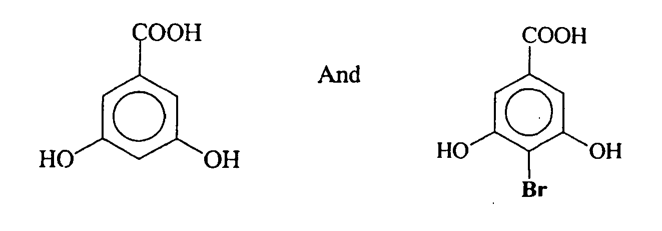

Synthesis process includes the hydroxyl methylation, ester reduction, alcohol oxidation, witting reaction( or Homer reaction or Horner-Emmons-Wadsworth reaction) and demethylation. The synthesis routes are well established and available in the art.

Additional stilbene derivatives may be obtained by standard esterification through the reaction of hydroxylated stilbene derivative and an acid or its derivative such as the corresponding salt, chloride and anhydride. This reaction is well known in the art. For example, an alcohol was added to a mixture of anhydride and pyridine at low temperature, and the mixture was left at room temperature for sufficient time to complete the reaction. After the reaction, general work up process that is known to the art gave the corresponding derivatives.

The inventive compounds of formula II may be produced with an appropriate oxidant, such as m-chloroperbenzoic acid, and hydrolysis of the corresponding compounds of formula 1. For example, 3,5-Dihydroxy-4-isopropyl-*trans*-stilbene epoxide can be synthesized from 3,5-dihydroxy-4-isopropyl-*trans*-stilbene compound **(9)** in Scheme 1 on next page by reacting with m-chloroperbenzoic acid (1.2 eq.) in CH₂Cl₂ at 0°C. After the reaction, the general work up process is known to the art. The compounds of the present invention may alternatively be prepared from different routes reported in the literature and are incoporated herein as references. (Eicher, T.; Tiefensee, K.; Donig, R. and Pick, R. Synthesis 1991, 98-102; Krow, G. R.; Miles, W. H.; Smiley, P. M.; Lester, W. S. and Kim, Y. J. J. Org. Chem. 1992, 57, 4040-4043; Seguineru, P. and Villieras Tetrahedron Letters 1988, 29, 477-480; Thakkar, K.; Geahlen, R. L. and Cushman, M. J. Med. Chem. 1993, 36, 2950-2955; Bezou, P.; Hilberer, A. and Hadziioannou, G. Synthesis 1996, 449-451). These methods are either using complex catalyst (Krow, G. R.; Miles, W. H.; Smiley, P. M.; Lester, W. S. and Kim, Y. J. J. Org. Chem. 1992, 57, 4040-4043) or involving many steps (Eicher, T.; Tiefensee, K.; Donig, R. and Pick, R. Synthesis 1991, 98-102).

### (a) Schemes of Synthesis

Compounds of the present inventions were synthesized from commercially available 3,4-dihydroxybenzoic acid **(1)** and 4-bromo-3,4-dihydroxybenzoic acid **(10)** following the route, outlined in **Scheme 1 or 2:**

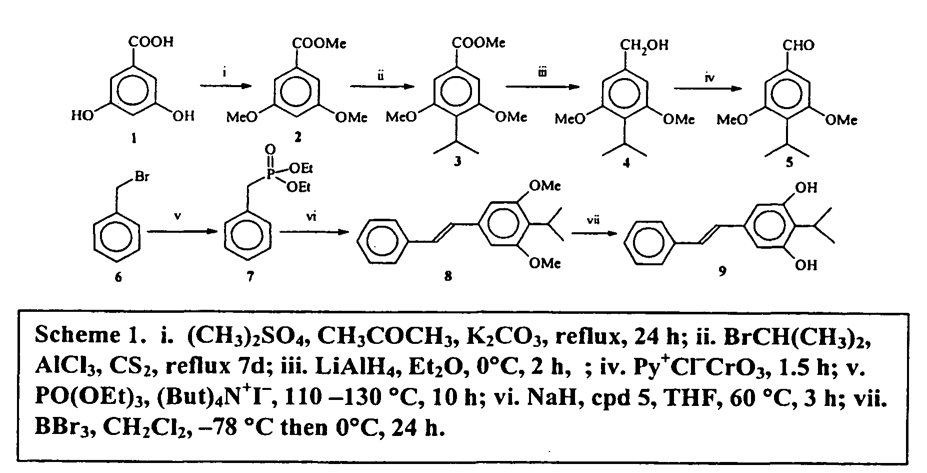

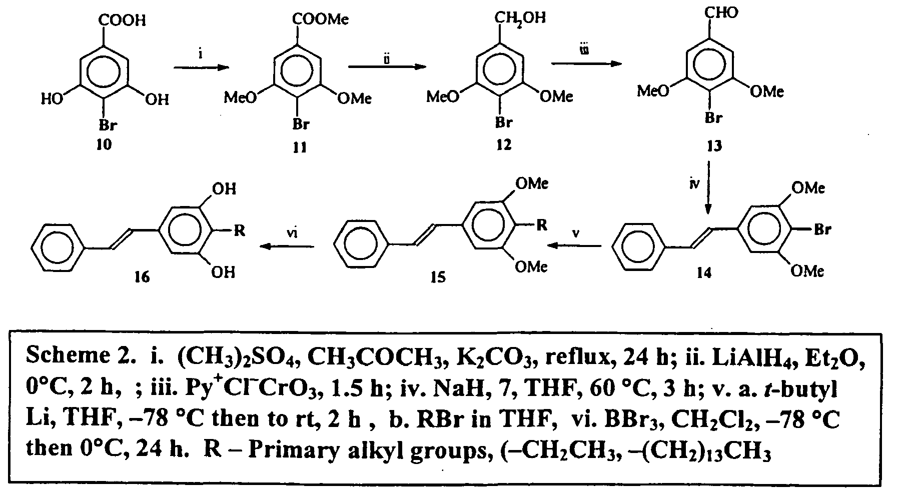

Procedure A (Methylation). Alcohol or acid (1 g, 1 eq) was added to a well stirred acetone solution (100 ml) containing dimethyl sulfate (2 eq. for each hydroxyl or carboxylic group) and K₂CO₃ (5 eq. for each hydroxyl or carboxylic group). This solution was refluxed for 12 hours. After filtration, solvent acetone was evaporated under reduced pressure, the residue was dissolved in EtOAc (50 mL). The EtOAc solution was washed with water (50 mL x 2), saturated aqueous NaCl (50 mL), dried over Na₂SO₄, evaporated under reduced pressure to offer syrup which was purified by silica column chromatography (Hexanes/EtOAc = 4: 1). When PO(OEt)3 is substituted with triphenylphosphate,cis-derivatives are synthesized.

Procedure B (Reduction of methyl ester to alcohol). LiAlH₄ (1.5 eq.) was added slowly with stir to the methyl ester (1 g, 1 eq) in anhydrous diethyl ether (100 mL) at 0 °C. After 30 min, water (5 mL) was added slowly to the mixture to quench excess LiAlH₄, and the mixture was acidified with 10 % HCl (aq). The organic layer was washed with water (50 mL x 2), saturated NaHCO₃ (50 mL), dried over Na₂SO₄ and the solvent was evaporated under reduced pressure to offer syrup which was crystallized from EtOH/hexanes.

Procedure C (Oxidation of primary alcohol to aldehyde). Alcohol (1g, 1 eq) in dichloromethane (10 mL) was added to a well stirred anhydrous dichloromethane (50 mL) containing suspended pyridinium chlorochromate (1.5 eq. to each hydroxyl group). After 90 min, diethyl ether (100 mL) was added, the supernatant was decanted and the black gummy syrup was washed with dry ether (20 mL x 3) and became a black solid. The combined organic solution was passed through a short pad of Florisol and the solvent was removed by rotary evaporation offering syrup that was then crystallized from EtOH/hexane.

Procedure D (Wittig Reaction). NaH (2 eq.) was added to a well stirred diethyl benzylphosphonate ester (7) (1.5 eq.) in dry THF (25 mL) for 60 min at 0 °C. Aldehyde (1 g, 1 eq) in THF (2 mL) was added slowly to the mixture, and the reaction mixture was allowed to react for 3 hours at 50 °C. After cooling down to room temperature, the mixture was poured onto ice, followed by addition of 2M HCl (5 mL). The mixture was extracted with EtOAc (20 mL x 3), the combined organic layer was then washed with water (25 mL x 2), saturated NaCl (25 mL) and dried over anhydrous magnesium sulfate. After filtration, the solvent was evaporated under reduced pressure, the resulting syrup was purified by silica column chromatographyy (Pet ether/ether = 8:1).

Procedure E (Demethylation): BBr₃ (4 mL, 1 M in CH₂Cl₂) in 10 mL of dry CH₂Cl₂ was added dropwise into methylated stilbene (1 g, 1 eq) in dry CH₂Cl₂ (5 mL) at - 78 °C, and left at room temperature overnight. The mixture was then poured on the ice and the organic layer was separated and aqueous layer was extracted with CH₂Cl₂ (20 mL x 2). The combined organic layer was washed with saturated NaCl, dried over anhydrous sodium sulfate and evaporated under reduced pressure to dryness.

Procedure F (Acetylation). Alcohol (1 g, 1 eq) was added to an ice-cold mixture of acetic anhydride/pyridine = 1:1 (v/v) (5 mL), and the mixture was left at room temperature overnight. After the reaction, EtOAc (25 mL) was added to the mixture, the EtOAc was washed with ice-cold water (25 mL), ice-cold 10 % HCl (25 mL x 2), water (25 mL x 2), saturated aqueous NaHCO₃ (25 mL), and dried over anhydrous Na₂SO₄. After removal of the solvent the product was purified by silica column chromatographyy (Hexanes/EtOAc = 8: 1).

### (c) Use of Synthesis Scheme 1 to make 3,5-Dihydroxy-4-isopropyl-trans-stilbene and its derivatives.

(i) Methyl 3,5-dimethoxy-benzoate **(compound 2** in Scheme 1) (Procedure A): the crude product (syrup) was crystallized from EtOH / Hexanes to give pure compound **2** (Scheme 1) (- 90 %). Mp: 110-113 °C. NMR (100 MHz, CDCl₃) δ 4.20 (s, 6 H, OCH₃), 4.60 (s, 3 H, COOCH₃), 6.30 (t, 1 H, J_{4,2} = 2.2 Hz, H-4), 7.20 (d, 2H, H-2,6).
(ii) Methyl 3,5-dimethoxy-4-isopropyl-benzoate **(compound 3** in Scheme 1): anhydrous AlCl₃ (0.85 g) was added to dry CS₂ (100 mL) containing methyl 3,5-dimethoxy-benzoate **(compound 2** in Scheme 1) (0.86 g) and 2-bromopropane (0.61 mL, 1.1 eq.). This solution was heated to reflux for 7 days. The mixture was filtered, washed with water (100 mL x 2), saturated NaHCO₃ (100 mL) and saturated NaCl (100 mL), dried over Na₂SO₄. After filtration and removal **of the** solvent, the crude product was purified by column chromatographyy (EtOAc/Hexanes = 4:1) to give methyl 3,5-dimethoxy-4-isopropyl-benzoate **(compound 3)** (0.69 g, 66 %) which was crystallized from EtOH/Hexanes. NMR (100 MHz, CDCl₃) δ 1.61 (d, 6 H, J_{1',2'} = 7.1 Hz, H-2'), 3.66 (hept, 1 H, H-1'), 3.88 (s, 6 H, OCH₃), 3.94 (s, 3 H, COOCH₃), 7.25 (s, 2 H, H-2,6).
(iii) 3,5-Dimethoxy-4-isopropyl-benzyl alcohol **(compound 4** in Scheme 1) (Procedure B): The crude product (syrup) was crystallized from EtOAc/Hexanes to give compound **4** (85 % yield). NMR (100 MHz, CDCl₃) δ 1.31 (d, 6 H, J_{1',2'} = 7.1 Hz, H-2'), 3.61 (hept, 1 H, H-1'), 3.84 (s, 6 H, OCH₃), 4.68 (s, 1 H, H-OH), 6.60 (s, 2 H, H-2,6).
(iv) 3,5-Dimethoxy-4-isopropyl-benzaldehyde **(compound 5** in Scheme 1) (Procedure C): The resulting residue was crystallized from EtOH to give pure compound **5** (80 % yield). NMR (100 MHz, CDCl₃) δ 1.31 (d, 6 H, J_{1',2'} = 7.1 Hz, H-2'), 3.61 (hept, 1 H, H-1'), 3.84 (s, 6 H, OCH₃), 4.68 (s, 1 H, H-OH), 6.60 (s, 2 H, H-2,6).
(v) Diethyl benzylphosphonate ester **(compound 7** in Scheme 1): Triethyl phosphite (3.2 mL, 1.5 eq.) was added to the benzyl bromide (2.11 g) containing a catalytic amount of tetrabutylammonium iodide, the mixture was heated to 110-130 °C overnight. Excess triethyl phosphite was removed by heating the solution for 1 hour at 100 °C under vacuum (15 mm Hg) to yield compound **7** quantitatively. NMR (100 MHz, CDCl₃) δ 1.21 (t, 6 H, J_{2',1'}= 7.1 Hz, H-2'), 3.02 (s, 1 H, H-α), 3.24 (s, 1 H, H-β), 3.98 (quint, 2 H, H-1'), 7.27 (s, 5 H, H-ArH).
(vi) 3,5-Dimethoxy-4-isopropyl-*trans*-stilbene **(compound 8** in Scheme 1) (Procedure D): The product was purified by column chromatography (Et₂O/Hexanes = 1:8) and crystallized from ether/hexanes to give pure compound **8** (70 % yield). Mp: 64-66 °C. NMR (400 MHz, CDCl₃) δ 1.28 (d, 6 H, J_{Me,CH}= 7.0 Hz, CH₃), 3.58 (hept, 1 H, -CH-), 3.85 (s, 6 H, H-OCH₃), 6.69 (s, 2 H, H-2, 6), 7.05 (s, 2 H, =CH), 7.25 (m, 1 H, H-4'), 7.35 (m, 2 H, H-3',5'), 7.25 (m, H-2',6').
(vii) 3,5-Dihydroxy-4-isopropyl-*trans*-stilbene **(compound 9** in Scheme 1) (Procedure E): The product was purified by column chromatography (EtOAc/Hexanes) and give desired compound **9** (95 % yield). Mp: 140-142 °C. NMR (400 MHz, CDCl₃) δ 1.38 (d, 6 H, J_{α,β} = 7.3 Hz, CH₃), 3.46 (hept, 1 H, -CH-), 4.80 (s, 2 H, H-OH), 6.50 (s, 2 H, H-2, 6), 6.92 (d, 1 H, J_{A,B} = 16.2 Hz, H- =CH_{A}), 6.97 (d, 1 H, H- =CH_{B}), 7.25 (m, 1 H, H-4'), 7.34 (m, 2 H, H-3',5'), 7.52 (m, 2 H, H-2',6').

### (d) Use of Scheme 2 to form additional 4-substituted 3,5-dihydroxy-trans-stilbenes and their derivatives.

(viii) Methyl 3,5-dimethoxy-4-bromo-benzoate **(compound 11** in Scheme 2) (Procedure A). The crude product (95 %) was crystallized from EtOH/ Hexanes. Mp: 119-124 °C. NMR (100 MHz, CDCl₃) δ 3.96 (s, 3 H, COOCH₃), 3.99 (s, 6 H, OCH₃), 7.28 (s, 2 H, H-2, 6).
(ix) 3,5-Dimethoxy-4-bromo-benzyl alcohol **(compound 12** in Scheme 2) (Procedure B). The crude product (85 % of yield) was crystallized from EtOH/Hexanes.Mp: 84-95 °C. NMR (100 MHz, CDCl₃) δ 1.95 (s, 1 H, OH), 3.93 (s, 6 H, OCH₃), 4.69 (s, 1 H, CH₂), 6.61 (s, 2H, H-2,6).
(x) 3,5-Dimethoxy-4-bromo-benzaldehyde **(compound 13** in Scheme 2) (Procedure C). The crude product (75 %) was crystallized from EtOH/Hexanes. Mp: 110-113 °C. NMR (100 MHz, CDCl₃) δ 4.02 (s, 6 H, OCH₃). 7.11 (s, 2H, H-2,6), 9.97 (s, 1 H, CHO).
(xi) 3,5-Dimethoxy-4-bromo-*trans*-stilbene **(compound 14** in Scheme 2) (Procedure D). The crude product was purified by column chromatography (Pet ether/Ether = 8: 1) in 70 % and crystallized from ether/hexanes. Mp: 149-152 °C. NMR (400 MHz, CDCl₃) δ 3.96 (s, 6 H, 2 x OCH₃), 6.72 (s, 2 H, H-2, 6), 7.06 (d, 1 H, J_{A,B}= 16.2 Hz, H-=CH_{A}), 7.11 (d, 1 H, H- =CH_{B}), 7.28 (m, 1H, H-4'), 7.37 (m, 2 H, H-3',5'), 7.55 (m, 2 H, H-2',6').
(xii) 4-Bromo-3,5-Hydroxy- -*trans*-stilbene **(compound 16** in Scheme 2) (Procedure E): The crude product was purified by column chromatography (Pet ether/Ether = 4: 1) in 90 % and crystallized from Ether/Hexanes. Mp: 150-152 °C. NMR (100 MHz, CDCl₃) δ 5.39 (s, 2 H, 2 x OH), 6.81 (s, 2 H, ArH-2, 6), 7.06 (d, 1 H, J_{A,B} = 16.2 Hz, H-=CH_{A}), 7.11 (d, 1 H, H- =CH_{B}), 7.28 (m, 1 H, H-4'), 7.37 (m, 2 H, H-3',5'), 7.55 (m, 2 H, H-2',6').
(xiii) 3,5-Dimethoxy-4-ethyl-*trans*-stilbene **(compound 15a** in Scheme 2): *t-*butyl Li (1.1 mL, 1M in THF) was added at - 78 °C to a THF solution (10 mL) containing 3,5-dimethoxy-4-bromo-*trans-*stilbene (0.53 g). After addition, the solution was slowly heated to reflux for 30 min and then cooled down to - 78 °C. Ethyl iodide (1.2 eq, 0.265 mL) was added to the solution at - 78 °C. After reaction finished, water (10 mL) was added dropwise to the mixture, THF was evaporated under reduced pressure. The mixture was extracted with CH₂Cl₂ (5 mL x 3), the combined organic layer was dried over anhydrous magnesium sulfate, and remove under reduced pressure. The product mixture was purified by column chromatography (ether/pet ether = 1:8) and gave 3,5-dimethoxy-4-ethyl-*trans*-stilbene (14a) (70%) and 3,5-dimethoxy-*trans*-stilbene (30 %) due to the moisture. Mp: 70-73 °C. NMR (400 MHz, CDCl₃) δ 1.12 (t, 6 H, J_{Me,HCH} = 7.2 Hz, CH₃), 2.70 (q, 2 H, -CH₂-), 3.91 (s, 6 H, OCH₃), 6.74 (s, 2 H, H-2, 6), 7.07 (s, 2 H, 2 x =CH-), 7.26 (m, I H, H-4'), 7.36 (m, 2 H, H-3',5'), 7:52 (m, 2 H, H-2',6').
(xiv) 3,5-Dihydroxy-4-ethyl-*trans*-stilbene **(compound 16a** in Scheme 2) (Procedure E): After column chromatography (ether/pet ether =8:1) the product was obtained in 91 % of yield and crystallized from ether/hexanes. Mp: 143-146 °C. NMR (100 MHz, CDCl₃) 5 1.22 (t, 6 H, J_{α,β} = 7.5 Hz, 2 x CH₃), 2.70 (q, 2 H, CH₂), 4.81 (s, 2 H, 2 x OH), 6.60 (s, 2 H, H-2, 6), 7.00 (s, 2 H, 2 x =CH-), 7.26 (m, I H, H-4'), 7.36 (m, 2 H, H-3',5'), 7.52 (m, 2 H, H-2',6').
(xv) 3,5-Dimethoxy-4-myristyl-*trans*-stilbene **(compound 15b** in Scheme 2). Procedure and work up are the same as compound **14** [see (xi) above]. Mp: 68-70 °C. NMR (100 MHz, CDCl₃) δ 0.91 (m, 6 H, 2 x CH₃), 1.29 (m, 22 H, 10 x CH₂), 2.65 (m, 2 H, CH₂), 3.90 (s, 6 H, 2 x OCH₃), 6.73 (s, 2 H, H-2, 6), 7.10 (s, 2 H, H- =CH), 7.26 (m, 1 H, H-4'), 7.36 (m, 2 H, H-3',5'), 7.52 (m, 2 H, H-2',6').
(xvi) 3,5-Dihydroxy-4-myristyl -*trans*-stilbene **(compound 16b** in Scheme 2) (Procedure E): After column chromatography (ether/pet ether =8:1) the product was obtained in 91 % of yield and crystallized from ether/hexanes: Mp: 125-128 °C. NMR (100 MHz, CDCl₃) δ 0.95 (m, 6 H, 2 x CH₃), 1.30 (m, 22 H, 10 x CH₂), 2.65 (m, 2 H, CH₂), 4.80 (s, 2 H, 2 x OH), 6.60 (s, 2 H, H-2, 6), 7.00 (s, 2 H, 2 x =CH-), 7.26 (m, 1 H, H-4'), 7.36 (m, 2 H, H-3',5'), 7.52 (m, 2 H, H-2',6').

### (e) Examples of additional hydroxy-trans-stilbene derivatives.

(xvii) 3,5-Di-acetoxy-4-isopropyl-*trans*-stilbene (Procedure E): The desired product was obtained in quantitative yield from 3,5-dihydroxy-4-isopropyl-trans-stilbene following procedure F and crystallized from ether/hexanes. Mp: 125-128 °C. NMR (400 MHz, CDCl₃) δ 1.25, 1.27 (s, 6 H, 2 x CH₃), 2.35 (s, 6 H, 2 x COCH₃), 3.08 (heptet, 1H, CH), 6.99, 7.02 (s, 2 H, H-2,6), 7.06 (s, 2 H, CH=CH), 7.26 (m, 1 H, H-4'), 7.36 (m, 2 H, H-3',5'), 7.52 (m, 2 H, H-2',6'). [
(xviii) 3,5-Di-chloroacetoxy-4-isopropyl-*trans*-stilbene: Triethyl amine (2 eq. for each OH group) was added to the mixture of 3,5-dihydroxy-4-isopropyl-*trans-*stilbene (143 mg) and chloroacetic anhydride (4 eq.) in ether (5 mL) at room temperature and left overnight. After evaporation of the solvent, the product was purified by silica column chromatography (EtOAc/Hexanes = 1:8) to give pure product that was crystallized from ether/hexanes (167 mg, 72 %). Mp: 83-85 °C. NMR (400 MHz, CDCl₃) δ 1.26, 1.33 (s, 6 H, 2 x CH₃), 3.08 (hept, 1H, CH), 4.39 (s, 4 H, ClCH₂CO), 6.99, 7.02 (s, 2 H, H-2,6), 7.06 (s, 2 H, CH=CH), 7.26 (m, I H, H-4'), 7.36 (m, 2 H, H-3',5'), 7.52 (m, 2 H, H-2',6').
(xix) 3,4',5-Tri-acetoxy-*trans*-stilbene (Procedure E): Following the Procedure F, the desired product was obtained quantitatively from 3,4'-5-trihydroxy-trans-stilbene (100 % yield). Mp: 113-116 °C. NMR (400 MHz, CDCl₃) δ 2.30 - 2.35 (s, 9 H, 3 x COCH₃), 6.82 (t, 1 H,J_{4,2} = J_{4,6} =2.5 Hz, H-4), 6.99(d, 1 H, J_{A,B} = 16.2 Hz, =CH_{A}), 7.04 (d, 1 H, =CH_{B}), 7.09 (m, 1 H, H_{AA'}--3',5' ), 7.12 (d, 1 H, H-2,6), 7.49 (m, 1H, H_{BB'}-2',6').
(xx) 3,4',5-Tri-acetoxy-*trans*-stilbene epxoide: m-chloroperbenzoic acid (1.2 eq.) was added to CH₂Cl₂ (1 mL) containing 3,4',5-tri-acetoxy-*trans*-stilbene (24 mg) at 0 °C, until TLC showed the disappearance of the starting material (∼ 3 hours). This solution was than washed with water ( 1 mL x 2), saturated NaHCO₃ (1 mL), saturated NaCl (mL) and dried over MgSO₄. After filtration and removal of the solvent, the syrup was purified by silica column chromatography (Hexanes/Ether = 8:1) to give pure compound which was crystallized from ether/hexanes (16 mg, 64 %). Mp: 133-137 °C. NMR (400 MHz, CDCl₃) δ 2.29 - 2.31 (s, 9 H, 3 x COCH₃), 3.82 (q, 2 H, J_{A,B} = 1.8 Hz, CH_{A}-CH_{B}), 6.89 (t, 1 H, J_{4,2} = J_{4,6} = 2.1 Hz, H-4), 6.97 (dd, 2 H, H-2,6), 7.10 (AA' of AA'BB', 1 H, H_{AA'}-3',5'), 7.34 (BB' of AA'BB', 1H, H_{BB}, -2',6').
(xxi) 3,4',5-Trimethoxy-*trans*-stilbene (Procedure A): The product was purified by silica column chromatography (EtOAc/Hexanes = 1:8) to give pure product (∼ 100 % yield) which was crystallized form ether/hexanes. Mp: 51-54 °C. NMR (400 MHz, CDCl₃) δ 3.83 (s, 9 H, 3 x OCH₃), 6.38 (t, 1 H, *J*_{4,3} = *J*_{4,5} = 4.6 Hz, H-4), 6.65 (d, 2 H, H-3,5), 6.88-6.91 (AA' of AA'BB', 2 H, H-2',6'), 6.91 (d, 1 H, J_{A,B} = 16.1 Hz, =CH_{A}), 7.04 (d, 1 H, H-=CH_{B}), 7.43-7.46 (BB' of AA'BB', 2 H, H-3',5').
(xxii) 3,4-Methylenoxy-*trans*-stilbene (Procedure D): Procedure D was used to synthesize the designed compound from 3,4-methylenoxynezaldehyde. The resulting syrup was purified by silica column chromatography (Ether/Hexanes = 1:8) to give pure product which was crystallized form ether/hexanes (75 %). Mp: 89-91 °C. NMR (400 MHz, CDCl₃) δ 5.99 (s, 2H, -CH₂-), 6.80 (d, 1 H, J_{5,6} = 8.1 Hz, H-5), 6.94 (d, 1 H, J_{AB} = 16.3 Hz, =CH_{A}), 6.95 (dd, 1 H, J_{6,2} = 0.4 Hz. H-6), 7.03 (d, 1 H, =CH_{B}), 7.08 (d, 1 H, H-2), 7.24 (m, I H, H-4'), 7.34 (m, 2 H, H-3',5'), 7.48 (m, 2 H, H-2',6').

### Example 2. Formulations of the inventive compounds.

### a. Ointment Formulation

| Ingredient | Amount |
|---|---|
| Active Ingredient (compound of the invention) | 0.05-20.0 mg |
| Ethanol | 100 µl |
| Mineral Oil, USP | 50.0 mg |
| White Petrolatum, USP | to make1.0 g |

### Procedure

A weighed quantity of white petrolatum and mineral oil are heated to 65 °C and uniformly mixed. The mixture is cooled to 50-55 °C. with stirring. The stated active ingredient which has been dissolved in ethanol and milled is added to the above with stirring. The ointment is cooled to room temperature.

### b. Lotion formulation

| Ingredient | Amount |
|---|---|
| Active Ingredient (compound of the invention) | 0.05-20.0 mg |
| Ethanol | 100 µl |
| Micronized Aluminum Monostearate | 50.0mg |
| Isopropyl Myristate | to make 1.0 g |

### Procedure

Heat about 90% of required isopropyl myristate to 60°C. Add aluminum monostearate with stirring and maintain heat to dissolve aluminum monostearate. Add the stated active ingredient dissolved in ethanol in the remaining quantity of isopropyl myristate. Add with stirring the solution of the stated active ingredient to the thickened solution of aluminum monostearate in isopropylmyristate previously cooled to 45°C. The lotion is cooled to room temperature with agitation.

### c. Gel Formulation

| Ingredient | Amount |
|---|---|
| Active Ingredient (compound of the invention) | 0.05-20.0 mg |
| Ethanol | 100 µl |
| Polyethylenes and Copolymers (A-C8) | 100.0 mg |
| Light Mineral Oil, | to make 1.0 g |

### Procedure

Add a portion of mineral oil (about 90%) in a suitable vessel. Heat to about 80°C. Add polyethylene (A-C8) to the mineral oil. The mixture is agitated slowly while hot until all the polyethylene is dissolved. Cool the above mixture quickly by placing the vessel in a cooling bath of 10-15°C and resume the agitation at normal speed. Once the content of the vessel reaches approximately 45°C, add a mixture of the stated active ingredient which was dissolved in ethanol to the above polymer solution. Allow the mixture to air cool with slow agitation. This will result in a gel form.

### d. Cream

| Ingredient | Amount |
|---|---|
| Active Ingredient (compound of the invention) | 0.05-20.0 mg |
| Ethanol | 100 µl |
| Glaxal base cream | to make 1.0 g |

### Procedure

A weighed quantity of the stated active ingredient which has been dissolved in 100µl of ethanol and thoroughly mixed with the Glaxal base cream at room temperature.

### Example 3. Use an an Anti Psoriatic Agent

The antipsoriatic activity of the compounds of this invention can be determined by measurement of the effect of the test compound *in vivo* because there is no animal model suitable for the disease. The compounds were tested on volunteers. In these tests, representative compound of this invention, i.e., 3,5-dihydroxy-4-isopropylstilbene is active in reducing or eliminating psoriatic symptoms.

The compositions of the present invention comprise a compound of formula I or formula II in an antipsoriatic amount together with a suitable pharmaceutical carrier. An antipsoriatic amount is defined as the amount of compound necessary to cause remission of the psoriatic symptoms, i.e., the psoriatic lesions. In the usual course of therapy, the active compound is incorporated into an acceptable vehicle to form a composition for topical administration to the affected area, or into a form suitable for oral administration, such as tablets, capsules or pills. Compositions for topical application may be exemplified by ointments, creams, lotions, solutions, suspensions, aerosols, gels, shampoos, soaps or dusting powders. Such compositions will normally be based upon standard carriers such as pharmaceutically acceptable vegetable oils and gelatin, gums and petrolatum. Other ingredients to the compositions of the present invention may be preservatives, coloring, flavoring, sweetening, thickening, suspending, dispersing, emulsifying, swelling, stabilizing, and buffering agents as required by the specific formulation. Such compositions are envisioned to contain the active ingredient in a 0.01-10% by weight amount.

Compositions for oral administration, other than the dosage units mentioned above may be exemplified by lozenges, powders, granulates, solutions, suspensions, or elixirs.

The required daily dosage may be administered in single or divided doses. The exact dose to be administered will, of course, be dependent upon the particular compound employed, the age and weight of the subject and the patent's individual response. Based on animal testing and comparisons with known active agents, typical doses of the compounds of formula I for topical administration for the treatment of psoriasis, mycosis fungoides and vitiligo are contemplated to be in the range of 0.01-5mg/kg daily. This daily amount may be administered in single or divided doses.

The following examples describe in detail compounds and compositions illustrative of the present invention and methods which have been devised for their preparation. It will be apparent to those skilled in the art that many modifications, both of materials and methods, may be practiced without departing from the purpose and intent of this disclosure.

A 1% cream of 3,5-dihydroxy-4-isopropylstilbene was prepared for tests on volunteers. Two volunteers, each with a long psoriasis history, were recruited for the tests. Neither of them was on any medication a month before the initiation of the test.

Volunteer 1, Male, 48 years old with scalp psoriasis for more than 15 years. He had used various conventional drugs and treatments, include, steroids, phototherapy and other medicine during the course of his illness. All of these treatments had no or very limited effect on this psoriasis.

Volunteer 2, Female, 24 years old with plaques on her back. She had used hydrocortisone before and stopped using it because of side effects.

The volunteers were treated once per day by applying the creams once per day on top of the affected area with the basic cream as the control. Two comparable body areas were chosen, and one was treated with the control and the other with the cream containing the compound of the invention.The cream of the invention contained 1% 3,5-dihydroxy-4-isopropylstilbene, and the other components in the cream described in Example 2(d). The control cream was identical except that it contained no 3,5-dihydroxy-4-isopropylstilbene.
Each cream was rubbed into the skin in the area to be treated until no more could be rubbed in.
Results: the inventive compound showed great efficacy on the volunteers treated in comparison with the untreated areas and before and after treatment.

In the case of volunteer 1, the area applied with the inventive compound started showing improvement in the inflammation and a decrease in proliferative cells three days after the treatment and completely clearance in 7 days. No change occurred in the condition of the area treated with the control cream.

In the case of volunteer 2, there was visible improvement in inflammation and in clearance of the proliferative cells three days after the treatment and significant improvement of the psoriasis were observed within seven days of treatment.

### Example 4 - Use as a Protein Kinase Inhibitor

The specific protein kinase activity of the compounds of the invention is shown by the fact that they are active in the in vitro test described here below.

### Test for Protein Kinease Inhibition

DNA-PK was purified from human placenta according to Chan, et al.(1996) and the in vitro assay of the inhibitory activity was done according as outlined below.

As a standard protocol, each was prepared in 100% DMSO and the assay was performed at the following conditions:

Mix 5ul of protein kinase solution, 5ul compound testing solution, 5µl substrate solution and 5 µl assay dilution buffer (20mM MOPS, pH7.2, 25mM β glycerophosphate, 20mM MgCl₂ 5mM EGTA, 2mM EDTA, 1mM DTT, 1mM sodium vanadate) in a 96 well microtitre plate. The reaction was started by adding 5 µl of radio-labelled ATP solution (250 µM ATP with 1 µCi of [γ³²P] ATP) to the reaction mixture and the plate was incubated at room temperature for 15 min. The reaction was stopped by spotting 10 µl of the reaction mixture onto a 96 well plate containing filter-paper discs. After washing the filter paper plate six times with 1% phosphoric acid, the plate was blow-dried and scintillation fluid was added into each well. The plate was then counted in a scintillation counter. IC50 values were calculated from triplicated samples. Table 1 shows the inhibitory activity of three representative compounds: 3,5-Dihydroxy-4-isopropyl-*trans*-stilbene epoxide (EP), 3,5-dihydroxy-4-isopropyl-*trans-*stilbene(CST) and 3,4',5-Tri-acetoxy-*trans*-stilbene(STA).

**Table 1. Protein kinase inhibitory activity**

| Kinase | IC50 (mM) | | |
|---|---|---|---|
| | EP | CST | STA |
| Lck | 0.10 | 0.27 | 0.54 |
| Ck2 | 0.31 | 0.13 | Nd* |
| DNA-Pk | 0.16 | 0.55 | Nd |
| Pim-1 | 0.16 | 0.18 | Nd |

| | | | |
|---|---|---|---|
| * Nd= not determined. | | | |

As can be appreciated from the activity data shown in Table 1, the compounds according to the invention are endowed with valuable properties of inhibiting protein kineases.

### Example 5 - Use as an Anti Inflammatory

3,5-dihydroxy-4-isopropylstilbene was tested for inhibitory activity against neutrophil activation by crystal and chemo attractants. Neutrophil activation plays a major role in inflammation.

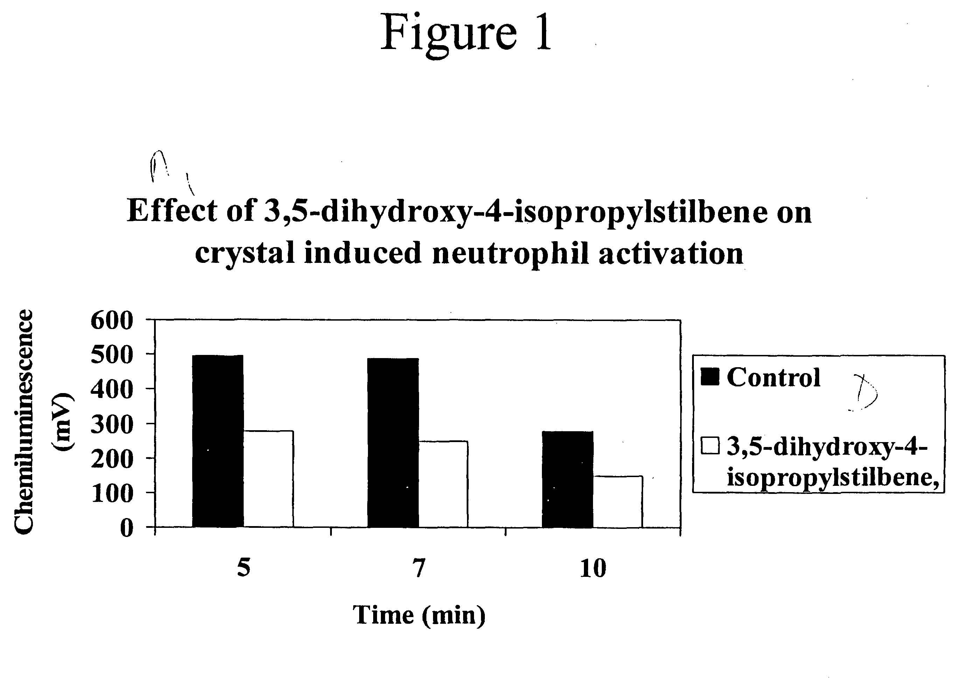

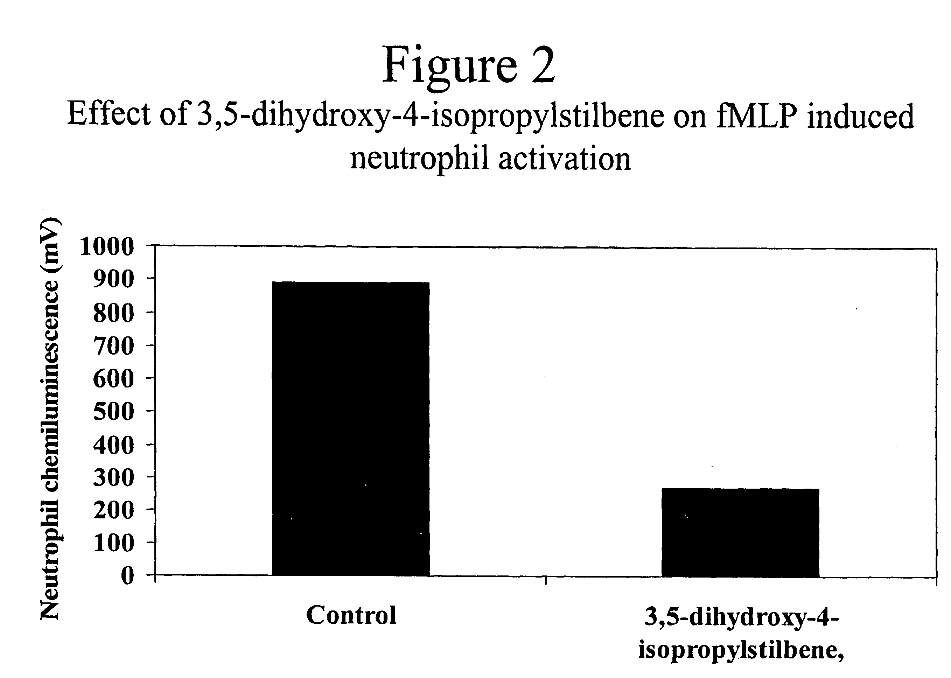

The test was done using an established protocol (Tudan. C. 1999. Bichem Pharmacol 58: 1869-1880). The results are shown in Figures 1 and 2.

Figure 1 shows that this compound shows significant inhibition of crystal-induced neutrophil activation.

Figure 2 shows that this compound shows significant inhibition of fMLP-induced neutrophil activation.

### REFERENCES

Chan, DW, Mody, CH, Ting, NS, Lees-Miller, SP, 1996, Purification and characterization of the double-stranded DNA-activated protein kinase DNA-PK from human placenta. Biochem- Cell Diol- 74(1): 67-73.
Cushman, M.; Nagarathnam, D.; Gopal, D.; He, H.-M; Lin, C.M.; Hamel, E. Synthesis and evaluation of analogues of (Z)-1-(4-Methoxyphenyl)-2-(3,4,5-Trimethoxyphenyl) ethene as potential cytotoxic and antimitotic agent. J. Med. Chem. 35:2293-2306, 1992.
Amson, R.; Sigaux, F.; Przedborski, S.; Flandrin, G.; Givol, D.; Telerman, A-: The human protooncogene product p33pim is expressed during fetal hematopoiesis and in diverse leukemias.Proc. Nat. Acad. Sci. 86: 8857-8861, 1989.
Ark, B.; Gummere, G.; Bennett, D.; Artzt, K. : Mapping of the Pim-1 oncogene in mouse t-haplotypes and its use to define the relative map positions of the telloci t-0(t-6) and t-w12 and the marker tf (tufted). Genomics 10: 385-389, 1991
Campbell MA, Sefton BM. Association between D-lymphocyto membrane immunoglobulin and multiple members of the Sre family of protein tyrosine kinases. Mol. Cell- Biol-, 12,2315 (1992).
Laird, P. W.; van der Lugt, N. M. T.; Clarke, A.; Domen, J.; Linders, K.; McWhir, J.; Berns, A.; Hooper, M. : In vivo analysis of Pim-1 deficiency. Nucleic Acids Res. 21: 4750-4755, 1993.
Saris, C. J. M.; Domen, J.; Berns, A. : The pim-1 oncogene encodes two related protein-serine/threonine kinases by alternative initiation at AUG and CUG. EMBO J. 10: 655-664, 1991.
VJ. Paul, S. Frautschv, W. Fenical, and K.H. Nealson,. Journal of Chemical Ecology, 7: 589-597 (1981),
K. Hu, J. Li, W. Wang, H. Wu, H. Lin and J. M. Webster, Canadian Journal of Microbiology. 44: 1072-1077 (1998).
Jang, M. L. Cai, G. Udeani, K. V. Slowing, C. F. Thomas, C. W. Beecher, H.S. Fong, N. R. Farnsworth, A. Kinghorn, R. Mehta, R. Moon and J. M. Pezzuto. Cancer Chemopreventive activity of resveratrol, a natural product derived from grapes. Science Vol. 275:218-220. (1997).
Garcia-Garcia, J. Micol V, de Godos A, Gomez-Fernandez. J. C. The cancer chemopreventive agent resveratrol is incorporated into model membranes and inhibits protein kinase C alpha activity. Arch Biochem Biophys 372(2):382-388. (1999).

## Claims

1. A compound of the Formula 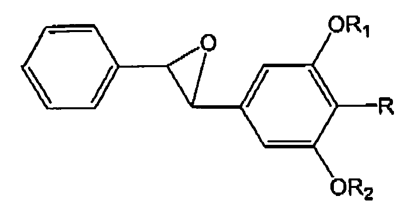 wherein
R is selected from the group consisting of
hydrogen;
halogen;
alkyl with carbon between 1 to 18;
alkenyl with carbon between 1 to 18;
alkoxy with carbon between 1 to 18;
R1 and R2 are independently selected from the group consisting of hydrogen;
alkyl with carbon between 1 to 18;
acyl with carbon between 1 to 18;
or pharmaceutically acceptable salts thereof.

2. A compound according to claim 1, which is 3,5-dihydroxy-4-isopropyl-stilbene-epoxide.

3. A pharmaceutical composition comprising a compound of Formula I or II: 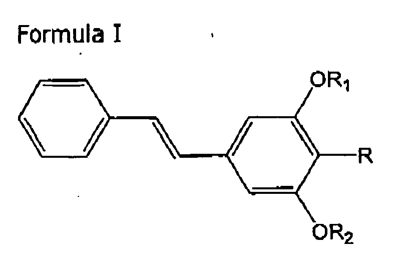 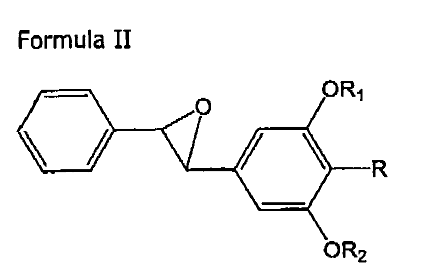 wherein
R is selected from the group consisting of
hydrogen (in the case of Formula II only);
halogen;
alkyl with carbon between 1 to 18;
alkenyl with carbon between 1 to 18;
alkoxy with carbon between 1 to 18 (in the case of Formula II only);
R1 and R2 are independently selected from the group consisting of
hydrogen;
alkyl with carbon between 1 to 18;
acyl with carbon between 1 to 18;
or pharmaceutically acceptable salts thereof.

4. The pharmaceutical composition according to claim 3, wherein in Formula I R1 and R2 are hydrogen and R is an alkyl group.

5. The pharmaceutical composition according to claim 3 or 4 comprising 3,5-dihydroxy-4-isopropylstilbene or 3, 5-diacetoxy-4-isopropylstilbene.

6. The pharmaceutical composition according to claim 3 or 4, comprising 3,5-dihydroxy-4-isoproyl-trans-stilbene-epoxide.

7. The pharmaceutical composition of any of claims 3-6 wherein it is useful for protein kinase inhibition.

8. The pharmaceutical composition of any of claims 3-6 wherein the compounds of Formula I are useful for treating psoriasis.

9. The pharmaceutical composition of any of claims 3-6 wherein the compounds of Formula I are useful for treating inflammation.

10. The pharmaceutical composition of claim 7, wherein the protein kinase is Lck, DNA-Pk, Pim-1 or Ck2.

## Patentansprüche

1. Verbindung der Formel 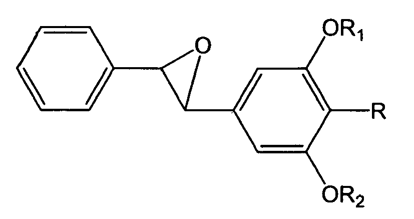 wobei
R ausgewählt ist aus der Gruppe bestehend aus
Wasserstoff;
Halogen;
Alkyl mit Kohlenstoffatomen zwischen 1 bis 18;
Alkenyl mit Kohlenstoffatomen zwischen 1 bis 18;
Alkoxy mit Kohlenstoffatomen zwischen 1 bis 18;
R1 und R2 unabhängig ausgewählt sind aus der Gruppe bestehend aus
Wasserstoff;
Alkyl mit Kohlenstoffatomen zwischen 1 bis 18;
Acyl mit Kohlenstoffatomen zwischen 1 bis 18;
oder deren pharmazeutisch annehmbaren Salzen.

2. Verbindung gemäß Anspruch 1, welche 3,5-Dihydroxy-4-isopropyl-stilben-epoxid ist.

3. Pharmazeutische Zusammensetzung, die eine Verbindung der Formel I oder II umfasst: 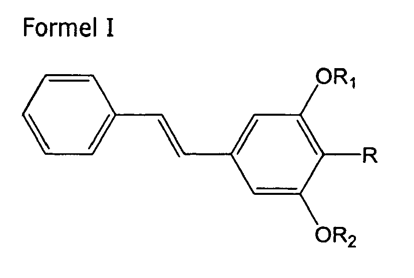 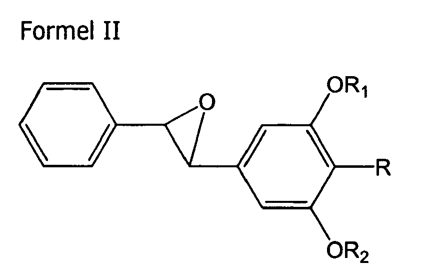 wobei
R ausgewählt ist aus der Gruppe bestehend aus
Wasserstoff (nur im Falle von Formel II);
Halogen;
Alkyl mit Kohlenstoffatomen zwischen 1 bis 18;
Alkenyl mit Kohlenstoffatomen zwischen 1 bis 18;
Alkoxy mit Kohlenstoffatomen zwischen 1 bis 18 (nur im Falle von Formel II); R1 und R2 unabhängig ausgewählt sind aus der Gruppe bestehend aus
Wasserstoff;
Alkyl mit Kohlenstoffatomen zwischen 1 bis 18;
Acyl mit Kohlenstoffatomen zwischen 1 bis 18;
oder deren pharmazeutisch annehmbaren Salzen.

4. Pharmazeutische Zusammensetzung gemäß Anspruch 3, wobei in Formel I R1 und R2 Wasserstoff sind und R eine Alkylgruppe ist.

5. Pharmazeutische Zusammensetzung gemäß Anspruch 3 oder 4, die 3,5-Dihydroxy-4-isopropylstilben oder 3,5-Diacetoxy-4-isopropylstilben umfassen.

6. Pharmazeutische Zusammensetzung gemäß Anspruch 3 oder 4, die 3,5-Dihydroxy-4-isoproyl-trans-stilben-epoxid umfassen.

7. Pharmazeutische Zusammensetzung gemäß einem der Ansprüche 3 bis 6, wobei diese zur Proteinkinasehemmung nützlich ist.

8. Pharmazeutische Zusammensetzung gemäß einem der Ansprüche 3 bis 6, wobei die Verbindungen der Formel I zur Behandlung von Psoriasis nützlich sind.

9. Pharmazeutische Zusammensetzung gemäß einem der Ansprüche 3 bis 6, wobei die Verbindungen der Formel I zur Behandlung von Entzündungen nützlich sind.

10. Pharmazeutische Zusammensetzung gemäß Anspruch 7, wobei die Proteinkinase Lck, DNA-Pk, Pim-1 oder Ck2 ist.

## Revendications

1. Composé de la Formule 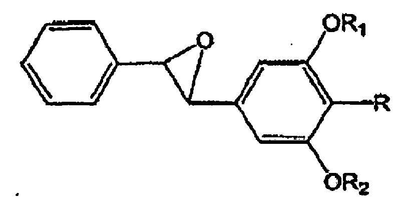 où
R est sélectionné parmi le groupe composé de
hydrogène;
halogène ;
alcoyle avec de 1 à 18 atomes de carbone ;
alkényle avec de 1 à 18 atomes de carbone ;
alcoxy avec de 1 à 18 atomes de carbone ;
R1 et R2 sont sélectionnés indépendamment du groupe composé de
hydrogène ;
alcoyle avec de 1 à 18 atomes de carbone ;
acyle avec de 1 à 18 atomes de carbone ;
ou des sels pharmaceutiquement acceptables de ces derniers.

2. Composé selon la revendication 1, qui est du 3,5-dihydroxy-4-isopropyl-stylbène-époxyde.

3. Composition pharmaceutique comprenant un composé de la Formule I ou II : 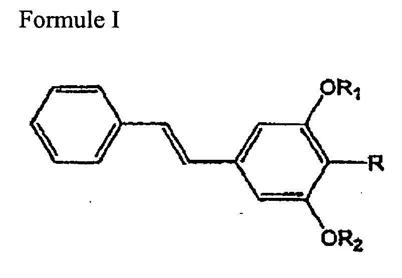 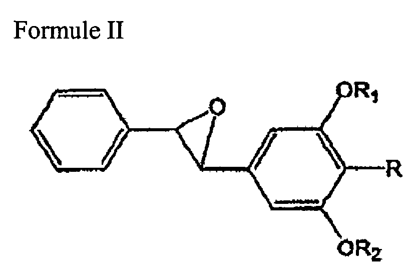 où
R est sélectionné parmi le groupe composé de
hydrogène (uniquement dans le cas de la Formule II) ;
halogène ;
alcoyle avec de 1 à 18 atomes de carbone ;
alkényle avec de 1 à 18 atomes de carbone ;
alcoxy avec de 1 à 18 atomes de carbone (uniquement dans le cas de la Formule II) ;
R1 et R2 sont sélectionnés indépendamment du groupe composé de
hydrogène ;
alcoyle avec de 1 à 18 atomes de carbone ;
acyle avec de 1 à 18 atomes de carbone ;
ou des sels pharmaceutiquement acceptables de ces derniers.

4. Composition pharmaceutique selon la revendication 3, dans laquelle, dans la Formule I, R1 et R2 sont hydrogène et R est un groupe alcoyle.

5. Composition pharmaceutique selon la revendication 3 ou 4, comprenant du 3,5-dihydroxy-4-isopropylstilbène ou 3,5-diacétoxy-isopropylstilbène.

6. Composition pharmaceutique selon la revendication 3 ou 4, comprenant du 3,5-dihydroxy-4-isopropyl-trans-stilbène-époxyde.

7. Composition pharmaceutique selon l'une quelconque des revendication 3 à 6, dans laquelle elle est utile pour l'inhibition de la proéine kinase.

8. Composition pharmaceutique selon l'une quelconque des revendication 3 à 6, dans laquelle les composés de la Formule I sont utiles pour le traitement du psoriasis.

9. Composition pharmaceutique selon l'une quelconque des revendication 3 à 6, dans laquelle les composés de la Formule I sont utiles pour le traitement de l'inflammation.

10. Composition pharmaceutique selon la revendication 3, dans laquelle la protéine kinase est Lck, DNA-Pk, Pim-1 ou Ck2.
